# EUROPEAN PATENT APPLICATION

(11) **EP 4 470 504 A1**
(43) Date of publication of application: **04.12.2024**
(21) Application number: 23769478.1
(22) Date of filing: 10.02.2023
(51) Int. Cl.: A61F 2/24

(54) **VALVE REPAIR SYSTEM AND COUPLING INSTRUMENT THEREOF**

(30) Priority: 18.03.2022 CN 202210267390
(71) Applicant: Hangzhou Dawneo Medical Technology Co., Ltd., Hangzhou, Zhejiang 311217 (CN)
(72) Inventor: MA, Kangling, Hangzhou, Zhejiang 311217 (CN); LIU, Xiang, Hangzhou, Zhejiang 311217 (CN); HE, Dong, Hangzhou, Zhejiang 311217 (CN); WEI, Yongqiang, Hangzhou, Zhejiang 311217 (CN)
(74) Representative: Nordmeyer, Philipp Werner
(86) International application number: PCT/CN2023/075405
(87) International publication number: WO 2023/173967

(57) **Abstract**

A valve repair system and a coupling instrument thereof are provided. The coupling instrument includes a prosthesis holder (90) and a balloon (100). The prosthesis holder (90) includes at least two prosthetic members (91) arranged circumferentially around its own axis (H). Proximal end of each prosthetic member (91) is fixed in position radially with respect to the prosthesis holder (90). The balloon (100) is inflatable to expand to actuate distal ends of the prosthetic members (91) to cause them to transition from a first deflected configuration into a second deflected configuration. With this design, when the prosthetic members (91) are clampingly assembled with the valve clip in the first deflected configuration, they are inwardly urged into coupling engagement with the valve clip. This enables the prosthesis holder (90) to be assembled with the valve clip without left-to-right asymmetry of the prosthetic members (91). Moreover, since the use of a pushing wire (04) and restriction rings (013) is dispensed with, the problem that the pushing wire (04) may experience great resistance or make a sound when it is being advanced or retracted is circumvented. Through using the balloon (100) to actuate the prosthetic members (91) into a different deflected configuration, it can be ensured that difficult separation of the prosthetic members (91) from the valve clip will not happen due to deflection of one or more of the prosthetic members (91) to an insufficiently large extent.

## Description

### TECHNICAL FIELD

The present invention relates to the field of medical devices and, in particular, to a valve repair system and a coupling instrument thereof.

### BACKGROUND

There are many causes of valvular regurgitation, such as valve prolapse, valve sclerosis in the elderly, rheumatic valvular disease in teenagers and young and middle-aged people, infectious valvulitis, enlargement of the heart, etc. Treatment is generally unnecessary for those with mild valvular regurgitation, who are asymptomatic or have only mild symptoms. They can work and live normally if no related discomfort is experienced. However, they are still recommended to receive regular examinations for preventing worsening of the condition. Patients with severe valvular regurgitation must receive medical care and appropriate treatment such as surgical repair of valvular regurgitation.

One strategy for surgical repair of valvular regurgitation is to fasten the heart valve with a valve clip. The surgical procedure involves advancing the valve clip into the atrium using a delivery system, followed by assembling the delivery system with the valve clip by manipulating both a coupling instrument and a pushing wire. Moreover, in the procedure, once the valve clip captures the target tissue, the delivery system is released from the valve clip.

Conventionally, the pushing wire is generally manipulated in concert with two restriction rings to couple and decouple the coupling instrument to and from the valve clip. Specifically, the coupling is simply accomplished by inserting the pushing wire through the two restriction rings. As a result, the two restriction rings is restricted and center-aligned with each other, retracting two clasps of the coupling instrument inwardly onto the valve clip. However, as the pushing wire is required to move in the restriction rings, its diameter has to be made slightly smaller than an opening diameter of the restriction rings. That is, clearances are left between the restriction rings and the pushing wire inserted therein. For this reason, the two restriction rings cannot be desirably center-aligned to an adequate degree of concentricity. The conventional coupling instrument for use with the valve clip suffers from at least the problems as follows:
Firstly, the two clasps of the coupling instrument tend to be asymmetric when the coupling instrument is assembled with the valve clip (i.e., the two clasps may form different angles with the axis of the pushing wire). Such asymmetry may hinder the release of the coupling instrument from the valve clip because one of the clasps may fail to deflect to a sufficiently large extent.
Secondly, the pushing wire tends to experience great resistance or make a sound when it is being advanced or retracted.
Thirdly, due to the clearances between the pushing wire and the restriction rings, in the assembled configuration, the clasps of the coupling instrument may slightly deflect but not tightly fit on the valve clip as desired.

Therefore, it would be desirable to have a coupling instrument which can be engaged and disengaged with and from an associated valve clip without suffering from the above three problems.

### SUMMARY

It is an objective of the present invention to provide a valve repair system and a coupling instrument thereof, which overcome the problems discussed above.

To this end, in a first aspect of the present invention, there is provided a coupling instrument used in a valve repair system. The coupling instrument includes a prosthesis holder and a balloon. The prosthesis holder includes at least two prosthetic members arranged circumferentially around its own axis. Each prosthetic member is proximally fixed in position radially with respect to the prosthesis holder. The balloon is inflatable to expand to actuate distal ends of the prosthetic members to cause them to transition from a first deflected configuration into a second deflected configuration. Each prosthetic member is configured in the first deflected configuration so that its distal end is spaced radially with respect to the prosthesis holder at a radial distance smaller than or equal to a radial distance at which its proximal end is spaced radially with respect to the prosthesis holder. Each prosthetic member is configured in the second deflected configuration so that its distal end is spaced radially with respect to the prosthesis holder at a radial distance greater than a radial distance at which its proximal end is spaced radially with respect to the prosthesis holder.

Optionally, the balloon may be disposed coaxial with the prosthesis holder.

Optionally, when the prosthetic members are in the first deflected configuration, the distal end of each prosthetic member may be spaced at an equal radial distance radially with respect to the prosthesis holder.

Optionally, the coupling instrument may include a push shaft extending along an axis of the prosthesis holder, which is inserted within the prosthesis holder, attached to the balloon and adapted to move axially to bring the balloon into, or remove it from, the prosthesis holder.

Optionally, the push shaft may be inserted through the balloon, wherein the balloon is sealingly attached both proximally and distally to the push shaft, and wherein the push shaft defines a transfer channel in communication with an interior of the balloon.

Optionally, the push shaft may be provided on its distal end portion with an external thread.

Optionally, the distal end of each prosthetic member may define a slot extending through the specific prosthetic member radially with respect to the prosthesis holder.

Optionally, the at least two prosthetic members may be symmetric about a center of the prosthesis holder.

Optionally, the prosthetic members may be made of a shape memory alloy, an initial configuration of the prosthetic members is configured as the first deflected configuration.

Optionally, the prosthesis holder may further include at least two insertion members, which are arranged circumferentially around and extend along the axis of the prosthesis holder.

In another aspect of the present invention, there is provided a valve repair system including a valve clip and the coupling instrument as defined above. The valve clip is detachably coupled to the coupling instrument. The valve clip includes: a spacer disposed coaxial with the prosthesis holder; a connector assembly which is movable relative to the spacer along an axis thereof; and two repair assemblies arranged symmetrically about a center of the spacer. Each repair assembly includes: an elastic spacing arm including a capture section and a support section, both extending along the axis of the spacer, wherein the spacer, the capture section and the support section are joined in order from proximal to distal, wherein a distal end of the capture section is joined to a proximal end of the support section at a joint defining a spacing portion, and wherein a distal end of the support section is connected to the connector assembly; a clasp which is provided on the capture section and adapted to clamp and fasten a predetermined target; and a compression ring including a proximal end connected to the spacing portion and a distal end connected to the connector assembly. The prosthesis holder is configured to be clampingly coupled to a proximal end of the spacer by deploying the prosthetic members into the first deflected configuration and to be separated from the proximal end of the spacer by deploying the prosthetic members into the second deflected configuration.

Optionally, the valve clip may include a proximal connector and a sealing gasket provided on the proximal connector, wherein the spacer is clampingly coupled to the prosthetic members by the proximal connector and the sealing gasket.

Optionally, the valve clip may further include a support body, which is disposed within the spacer around a distal end thereof and defines a first guide bore extending through the support body along the axis of the spacer, the first guide bore including a first bore section, a second bore section and a third bore section, which are joined in order from proximal to distal, both the first and third bore sections having a greater radial dimension than the second bore section, the radial dimension of the first bore section gradually decreasing from proximal to distal, the radial dimension of the third bore section gradually increasing from proximal to distal.

Optionally, the clasp may include a clasping sheet and a support sheet, the support sheet provided on the capture section, the clasping sheet elastically joined at one end to the support sheet so that the clasping sheet is elastically biased toward the support sheet.

Optionally, the clasping sheet may be provided with barbs including long barbs and short barbs, each long barb including a fixed portion, an intermediate portion and a tapered portion, which are joined in this order, the fixed portion connected to the clasping sheet.

Optionally, the tapered portion may be shaped as a quadrangular pyramid.

Optionally, the clasping sheet may be telescopic in its own direction of extension.

Optionally, the clasping sheet may include a number of telescopic sections, which are joined in the direction of extension of the clasping sheet so as to generally appear like waves.

In summary, the present invention provides a valve repair system and a coupling instrument thereof. The coupling instrument includes a prosthesis holder and a balloon. The prosthesis holder includes at least two prosthetic members, which are arranged circumferentially around an axis of the prosthesis holder and proximally fixed in position radially with respect to the prosthesis holder. The balloon can be inflated to expand to actuate distal ends of the prosthetic members to cause them to transition from a first deflected configuration into a second deflected configuration. In the first deflected configuration, each prosthetic member is configured so that its distal end is spaced radially with respect to the prosthesis holder at a radial distance smaller than or equal to a radial distance at which its proximal end is spaced radially with respect to the prosthesis holder. In the second deflected configuration, each prosthetic member is configured so that its distal end is spaced radially with respect to the prosthesis holder at a radial distance greater than a radial distance at which its proximal end is spaced radially with respect to the prosthesis holder.

Firstly, as the prosthesis holder is clampingly assembled with the valve clip by deploying the prosthetic members into the first deflected configuration, the attachment of the prosthetic members to the valve clip is accomplished by inward forces. This is advantageous over the prior art, in which, after a coupling instrument is assembled with a valve clip, clasps of the coupling instrument experience outward forces, necessitating engagement of a pushing wire with two restriction rings for preventing outward deflection of the clasps, because the inward forces enable the prosthesis holder to be assembled with the valve clip without left-to-right asymmetry of the prosthetic members. Accordingly, the prosthetic members can be preformed in the first deflected configuration and will not deflect at all after the prosthesis holder is coupled to the valve clip. That is, the prosthesis holder can be more stably coupled to the valve clip. Moreover, as the pushing wire and restriction rings are omitted, the problem that the pushing wire may experience great resistance or make a sound when it is being advanced or retracted is circumvented.

Secondly, according to the present invention, the balloon is used to actuate the prosthetic members of the prosthesis holder to cause them to transition from the first deflected configuration to the second deflected configuration. Since the balloon expands symmetrically, it can be ensured that equal angles between the individual prosthetic members and an axis of the prosthesis holder are maintained during their transitioning from the first deflected configuration to the second deflected configuration, which can prevent difficult separation from the valve clip which may happen when one or more of the prosthetic members deflect to an insufficiently large extent in the second deflected configuration.

### BRIEF DESCRIPTION OF THE DRAWINGS

Those of ordinary skill in the art would appreciate that the following drawings are presented to enable a better understanding of the present invention and do not limit the scope thereof in any sense, in which:
Fig. 1A is a schematic illustration of a coupling instrument;
Fig. 1B is a side view of Fig. 1A;
Fig. 1C schematically illustrates the coupling holder coupled to a valve clip;
Fig. 1D schematically illustrates the coupling holder coupled to a proximal connector;
Fig. 2 is a schematic illustration of a coupling holder according to an embodiment of the present invention, in which prosthetic members are in a second deflected configuration;
Fig. 3 is a schematic illustration of a prosthesis holder according to an embodiment of the present invention, in which prosthetic members are in a first deflected configuration;
Fig. 4 is a side view of Fig. 3;
Fig. 5 is a schematic illustration of a balloon according to an embodiment of the present invention, which is in an expanded configuration;
Fig. 6 is a side view of Fig. 5;
Fig. 7 is a schematic illustration of a push shaft according to an embodiment of the present invention;
Fig. 8 is a schematic illustration of a push shaft according to an embodiment of the present invention, with a balloon being provided thereon in a collapsed configuration;
Fig. 9 is a schematic illustration of a push shaft according to an embodiment of the present invention, with a balloon being provided thereon in an expanded configuration;
Fig. 10 shows a coupling holder assembled with a valve clip according to an embodiment of the present invention, with prosthetic members being in a first deflected configuration;
Fig. 11 is a side view of Fig. 10;
Fig. 12 shows a coupling holder separate from a valve clip according to an embodiment of the present invention, with the prosthetic members being in a second deflected configuration;
Fig. 13 is a side view of Fig. 12;
Fig. 14 is a schematic illustration of a proximal connector according to an embodiment of the present invention;
Fig. 15 is a schematic illustration of a sealing gasket according to an embodiment of the present invention;
Fig. 16 is an exploded view of a prosthesis holder, a sealing gasket and a proximal connector according to an embodiment of the present invention;
Fig. 17 is a schematic illustration of a valve clip according to an embodiment of the present invention, in which spacing arms are in a first configuration;
Figs. 18 and 19 are schematic illustrations of a valve clip according to an embodiment of the present invention, in which spacing arms are in a second configuration;
Figs. 20 and 21 are schematic illustrations of a valve clip according to an embodiment of the present invention, in which spacing arms are in a third configuration;
Fig. 22 schematically illustrates spacing arms in a third configuration and a spacer according to an embodiment of the present invention;
Fig. 23 is a cross-sectional view of a valve clip according to an embodiment of the present invention, taken along an axis of a spacer, in which spacing arms are in a third configuration;
Fig. 24 is a schematic illustration of a compression ring according to an embodiment of the present invention;
Fig. 25 is a side view of Fig. 24;
Figs. 26 and 27 are schematic illustrations of a clasp according to an embodiment of the present invention;
Fig. 28 is another schematic illustration of the clasp according to an embodiment of the present invention;
Fig. 29 is a schematic illustration of barbs on a clip according to an embodiment of the present invention;
Fig. 30 is a schematic illustration of a support body according to an embodiment of the present invention;
Fig. 31 is a cross-sectional view of Fig. 30 taken along line A-A;
Fig. 32 is a schematic illustration of a distal connector according to an embodiment of the present invention;
Fig. 33 is a schematic illustration of a guidewire connector according to an embodiment of the present invention;
Fig. 34 is a schematic illustration of a cap connector according to an embodiment of the present invention; and
Fig. 35 shows compression rings assembled with a distal connector according to an embodiment of the present invention.

### List of Reference Numerals

01 coupling holder; 011 clasp; 0110 slot; 012 insertion prong; 013 restriction ring; 02 spacer; 03 proximal connector; 031 first protrusion; 0310 insertion slot; 032 second protrusion; 04 pushing wire;
10 spacer; X connector assembly;
20 distal connector; 21 connector receptacle; 22 first engagement recesses;
30 repair assembly; 31 spacing arm; 311 capture section; 312 support section; 310 spacing portion; 32 clasp; 321 clasping sheet; 3211 elongate slot; 3212 control slot; 3213 telescopic section; 322 support sheet; 3221 transverse part; 3222 longitudinal part; 323 barb; 3231 long barb; 3232 short barb; 3233 fixed portion; 3234 intermediate portion; 3235 tapered portion; 33 compression ring; 331 compression hole; 332 second engagement recess;
40 proximal connector; 41 first extension; 42 second extension; 420 insertion slot;
50 sealing gasket; 51 third extension; 510 first slot; 52 second slot;
60 support body; 61 first guide bore; 611 first bore section; 612 second bore section; 613 third bore section; 62 support hole;
70 guidewire connector; 71 fourth extension; 710 second guide bore; 72 sixth extension; 73 notch;
80 cap connector; 81 fifth extension;
90 prosthesis holder; 91 prosthetic member; 910 prosthetic slot; 92 insertion member;
100 balloon;
110 push shaft; 111 transfer channel; 112 external thread;
120 control wire.

### DETAILED DESCRIPTION

Objectives, features and advantages of the present invention will become more apparent upon reading the following description with reference to the accompanying drawings, which illustrate particular embodiments thereof. Note that the figures are provided in a very simplified form not necessarily drawn to exact scale for the only purpose of helping to explain the disclosed embodiments in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents. As used herein, the term "or" is generally employed in the sense of "and/or", "some" of "at least one" and "at least two" of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two such items. The terms "one end" and "other end", as well as "proximal end" and "distal end", are used to generally refer to opposing ends including the opposing endpoints, rather than only to the endpoints. The terms "mount", "couple", "connect" and any variants thereof should be interpreted in a broad sense. For instance, a connection may be a permanent, detachable or integral connection, or a mechanical or electrical connection, or a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location relative to the other element, unless the context clearly dictates otherwise. Those of ordinary skill in the art can understand the specific meanings of the above-mentioned terms herein, depending on their context.

It is to be noted that, as used herein, the term "proximal end" generally refers to an end of a medical device closer to an operator who is operating it, while the term "distal end" generally refers to an end of the device that first enters a patient's body, during normal operation of the device.

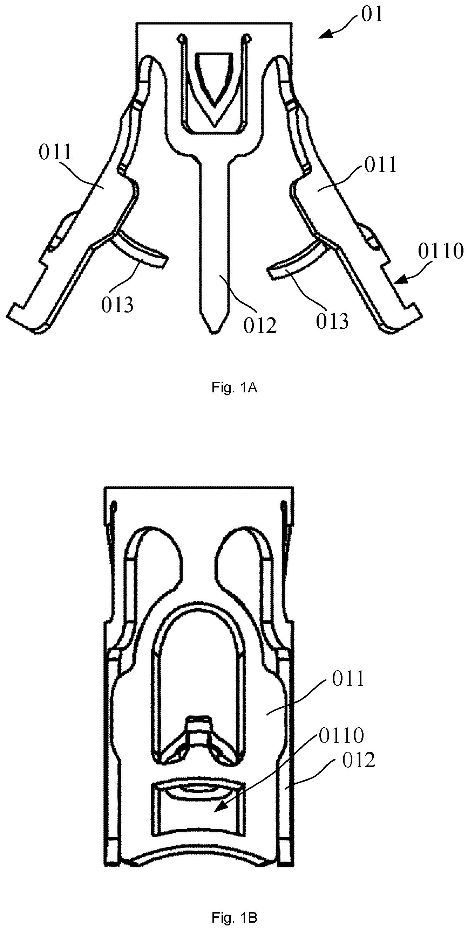

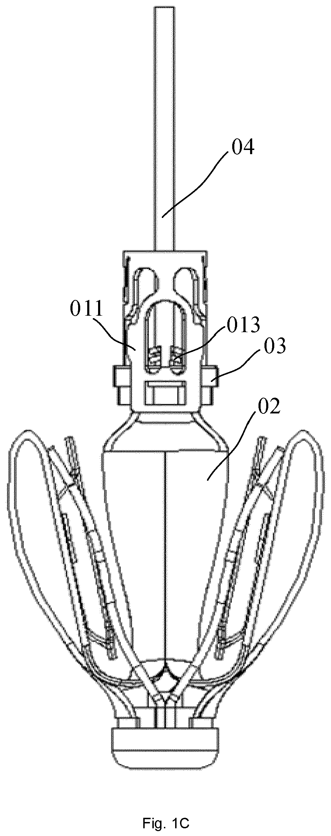

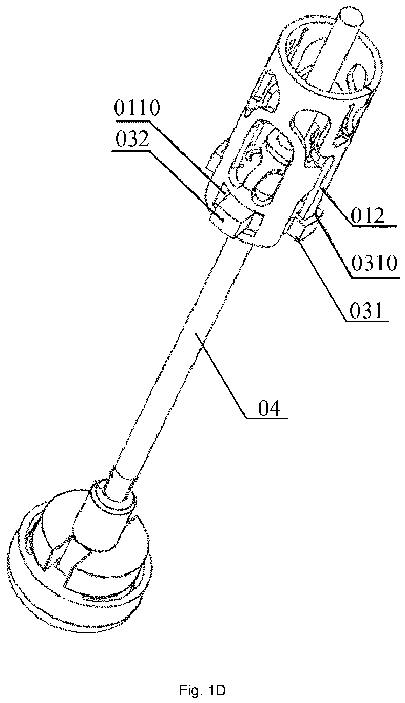

Fig. 1A is a schematic illustration of a coupling holder, and Fig. 1B is a side view of Fig. 1A. As shown in Figs. 1A and 1B, the coupling holder 01 generally includes two symmetrical clasps 011 and two symmetrical insertion prongs 012. The two clasps 011 are joined in a direction perpendicular to a direction in which the two insertion prongs 012 are joined. Each clasp 011 is provided on its inner side with a restriction ring 013. Each clasp 011 defines a through slot 0110. Fig. 1C schematically illustrates the coupling holder coupled to a valve clip, and Fig. 1D schematically illustrates the coupling holder coupled to a proximal connector. As shown in Figs. 1C and 1D, the valve clip includes a spacer 02 and a proximal connector 03 provided at a proximal end of the spacer 02. The proximal connector 03 has two outwardly extending first protrusions 031, the two first protrusions 031 are centrosymmetric about the center of the proximal connector 03, each first protrusion 031 defines a through insertion slots 0310. The proximal connector 03 also has two outwardly extending second protrusions 032.

Conventionally, before the coupling holder 01 is coupled to the valve clip, the coupling holder 01 assumes an open configuration in which the two clasps 011 deflect outwardly (see Fig. 1A). In order to assemble the coupling holder 01 with the valve clip, the insertion prongs 012 of the coupling holder 01 are inserted into the insertion slots 0310 of the first protrusions 031, and the two clasps 011 are then inwardly pressed to snap engage the second protrusions 032 of the proximal connector 03 with the slots 0110 of the clasps 011. Consequently, the two restriction rings 013 are substantially center-aligned with each other. Subsequently, a pushing wire 04 is introduced into the coupling holder 01, the pushing wire 04 passes through the two restriction rings 013. After that, it is passed into the spacer 02 and secured to a distal connector provided at a distal end of the spacer 02. In this way, under the action of the pushing wire passed through the two restriction rings 013, the clasps 011 cannot restore the open configuration shown in Fig. 1. As a result, the clasps 011 cannot separate from the second protrusions 032. In this way, the coupling holder 01 is coupled to the valve clip.

The coupling holder 01 can be decoupled from the valve clip simply by retracting the pushing wire 04 successively away from the spacer 02, the restriction rings 013 and the coupling holder 01. Once the pushing wire 04 no longer restricts the two restriction rings 013 and center-align them with each other, the clasps 011 deflect away from the proximal connector 03. Consequently, the second protrusions 032 are no longer received in the slots 0110, and the open configuration of Fig. 1A is restored. The coupling holder 01 can be then separated from the valve clip by removing the insertion prongs 012 from the insertion slots 0310.

Therefore, the coupling holder 01 relies simply on the pushing wire 04, which is passed through the two restriction rings 013 to center-align the two restriction rings 013 with each other, to inwardly gather the two clasps 011. However, as the pushing wire 04 is required to move in the restriction rings 013, the diameter of the pushing wire 04 has to be made slightly smaller than an opening diameter of the restriction rings 013. That is, there are clearances between the restriction rings 013 and the pushing wire 04 inserted therein. For this reason, the two restriction rings 013 cannot be desirably center-aligned to an adequate degree of concentricity. As a consequence, the two clasps 011 of the coupling holder 01 tend to be asymmetric when the coupling holder 01 is assembled with the valve clip (i.e., as viewed in the orientation of Fig. 1A, the clasps 011 may form different angles with the axis of the pushing wire 04, that is, the clasps 011 are left-to-right asymmetric with respect to the insertion prongs 012). Such asymmetry may make one of the clasps 011 difficult to deflect away from the respective second protrusion 032, eventually hindering the release of the coupling holder 01 from the valve clip. As another consequence, the pushing wire 04 tends to experience great resistance or make a sound when it is being advanced or retracted. As a further consequence, due to the clearances between the pushing wire 04 and the restriction rings 013, after being assembled, the clasps 011 of the coupling holder 01 may slightly deflect but not tightly fit on the valve clip as desired.

In view of the three problems described above, embodiments of the present invention provide valve repair systems and coupling instruments thereof.

The valve repair systems and coupling instruments will be described in detail below with reference to the accompanying drawings.

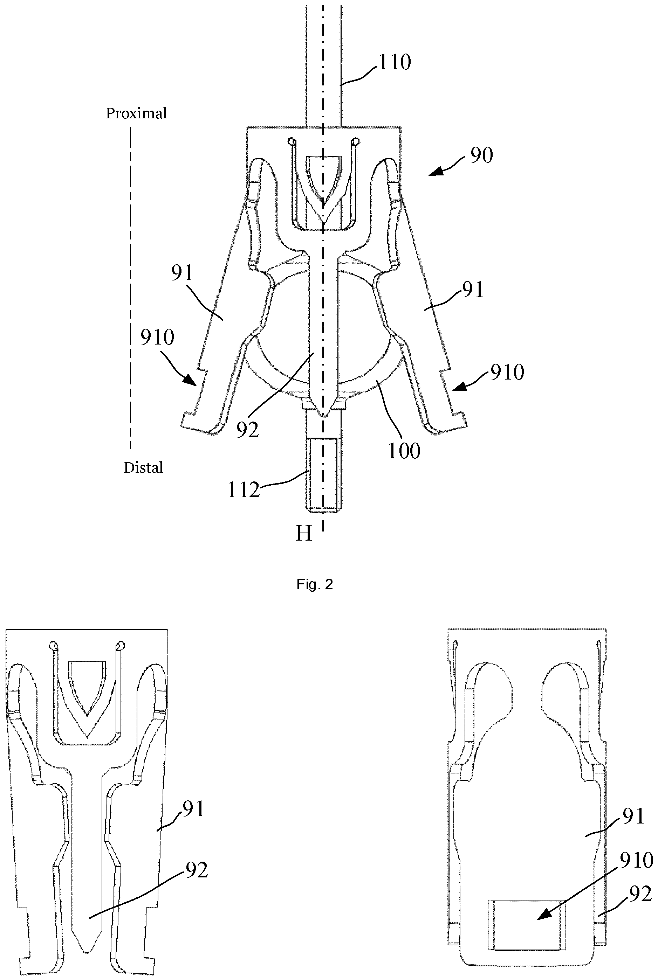

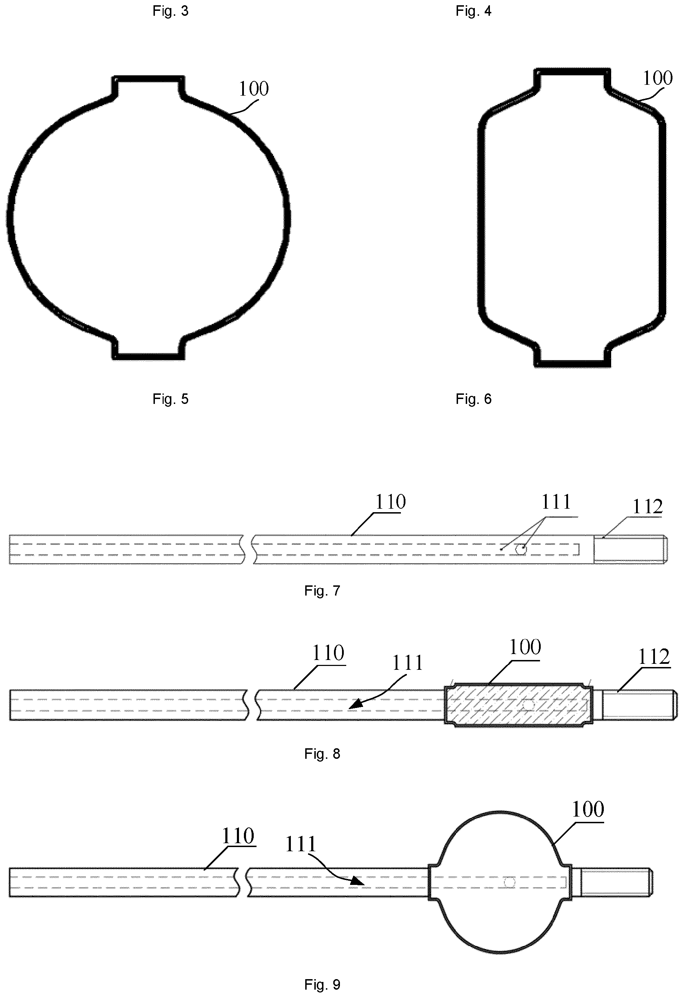

Fig. 2 is a schematic illustration of a coupling instrument according to an embodiment of the present invention, in which prosthetic members are in a second deflected configuration. Fig. 3 is a schematic illustration of a prosthesis holder according to an embodiment of the present invention, in which prosthetic members are in a first deflected configuration. As shown in Figs. 2 and 3, in an embodiment of the present invention, there is provided a coupling instrument including a prosthesis holder 90 and a balloon 100. The prosthesis holder 90 defines an axis denoted as H. Preferably, the balloon 100 is provided coaxial with the prosthesis holder 90. That is, an axis of the balloon 100 is coincident with the axis H. The prosthesis holder 90 includes at least two prosthetic members 91 arranged circumferentially around its axis (i.e., the axis H). The proximal end of each prosthetic member 91 is fixed radially with respect to the prosthesis holder 90. That is, the proximal end of each prosthetic member 91 is spaced at a fixed perpendicular distance from the axis H. The balloon 100 can expand to actuate distal ends of the prosthetic members 91 to cause them to transition from a first deflected configuration to a second deflected configuration. In the first deflected configuration, each prosthetic member 91 is configured so that its distal end is spaced radially with respect to the prosthesis holder 90 at a radial distance smaller than or equal to a radial distance at which its proximal end is spaced radially with respect to the prosthesis holder 90. In the second deflected configuration, each prosthetic member 91 is configured so that its distal end is spaced radially with respect to the prosthesis holder 90 at a radial distance greater than a radial distance at which its proximal end is spaced radially with respect to the prosthesis holder 90. Obviously, in the course of the prosthetic members 91 transitioning from the first deflected configuration to the second deflected configuration, their distal ends move outwardly (away from the axis H) radially with respect to the prosthesis holder 90. On the contrary, while the prosthetic members 91 are transitioning from the second deflected configuration to the first deflected configuration, their distal ends move inwardly (towards the axis H) radially with respect to the prosthesis holder 90. Moreover, when the balloon 100 is disposed within the prosthesis holder 90 and the balloon 100 is in an expanded configuration, the prosthetic members 91 will be maintained in the second deflected configuration by the balloon 100. When the balloon 100 is disposed outside of the prosthesis holder 90, or when the balloon 100 is disposed within the prosthesis holder 90 and the balloon 100 is disposed in a collapsed configuration, the prosthetic members 91 will not be restricted by the balloon 100, so that the prosthetic members 91 assume the first deflected configuration. Preferably, the at least two prosthetic members 91 are centrosymmetric about the center of the prosthesis holder 90. Preferably, when there are multiple prosthetic members 91, every two prosthetic members 91 may be pairwise centrosymmetric about the center of the prosthesis holder 90. Notably, here, by "centrosymmetric", it is intended to mean that the prosthetic members 91 are centrosymmetric in terms of position, but not that the prosthetic members 91 form symmetrical angles with the axis H. Accordingly, the centrosymmetry of the at least two prosthetic members 91 about the center of the prosthesis holder 90 can be construed as symmetry of the proximal ends of the prosthetic members 91 about the axis H of the prosthesis holder 90. It should be understood that the radial distance is measured diametrically or in a direction radiating from the center. A maximum radial distance is measured from the center to a circle defined by the prosthesis holder 90, i.e., as a radius of the circle. According to embodiments of the present invention, the proximal end of each prosthetic member 91 is spaced radially with respect to the prosthesis holder 90 at a radial distance which is equal to a perpendicular distance from the proximal end of the prosthetic member 91 to the axis H. Similarly, the distal end of each prosthetic member 91 is spaced radially with respect to the prosthesis holder 90 at a radial distance which is equal to a perpendicular distance from the distal end of the prosthetic member 91 to the axis H. It would be appreciated that when the prosthetic members 91 are configured in the first deflected configuration so that their distal ends are spaced radially with respect to the prosthesis holder 90 at radial distances equal to radial distances at which the proximal ends of the prosthetic member 91 are spaced radially with respect to the prosthesis holder 90, the prosthetic members 91 extend parallel to the axis H. Preferably, in the first deflected configuration, the distal end of each prosthetic member 91 is spaced radially with respect to the prosthesis holder 90 at an equal radial distance. That is, the distal end of each prosthetic member 91 is spaced at an equal perpendicular distance from the axis H.

In addition, the prosthetic members 91 may be originally formed in the first deflected configuration using a heat setting process. For example, each prosthetic member 91 may be made of a shape memory alloy, such as a nickel-titanium alloy, an initial configuration of the prosthetic members is configured as the first deflected configuration. Preferably, the remembered shape of each prosthetic member 91 is such that the distal end of the prosthetic member 91 is spaced radially with respect to the prosthesis holder 90 at a radial distance smaller than a radial distance, at which the proximal end of the prosthetic member 91 is spaced radially with respect to the prosthesis holder 90. As such, in the first deflected configuration, each prosthetic member 91 extends in a direction forming an angle with the axis H.

Fig. 4 is a side view of Fig. 3. Referring to Fig. 4, a distal end portion of the prosthetic member 91 defines a prosthetic slot 910, the prosthetic slot 910 extends through the prosthetic member 91 radially with respect to the prosthesis holder 90. Referring to Figs. 2 and 3, the prosthesis holder 90 further includes at least two insertion members 92 arranged circumferentially around its axis (i.e., the axis H). The insertion members 92 extend axially with respect to the prosthesis holder 90, i.e., the extension direction of the insertion members 92 is parallel to the axis H. Preferably, the at least two insertion members 92 are centrosymmetric about the center of the prosthesis holder 90. Preferably, the insertion members 92 are pairwise symmetrical about the axis H.

Fig. 5 is a schematic illustration of the balloon in the expanded configuration according to an embodiment of the present invention, and Fig. 6 is a side view of Fig. 5. The present invention is not limited to any particular shape of the balloon 100. For example, when in the expanded configuration, a middle portion of the balloon 100 may be spherical (see Fig. 5) or cylindrical. In some other embodiments, when in the expanded configuration, the middle portion of the balloon 100 may be ellipsoidal. Indeed, the balloon 100, when expanded, may assume any suitable shape, as long as it does not cause any interference within the prosthesis holder 90. Further, in the collapsed configuration, the balloon 100 is generally cylindrical.

Further, the balloon 100 may be inserted into the prosthesis holder 90 and then inflated and expanded to cause the prosthetic members 91 to transition from the first deflected configuration to the second deflected configuration. Moreover, the balloon 100 may be removed from the prosthesis holder 90 after it is deflated and collapsed back into the second deflected configuration. Fig. 7 is a schematic illustration of a push shaft according to an embodiment of the present invention. Fig. 8 is a schematic illustration of the push shaft according to an embodiment of the present invention, with the balloon being provided thereon in the collapsed configuration. Fig. 9 is a schematic illustration of the push shaft according to an embodiment of the present invention, with the balloon being provided thereon in the expanded configuration. Referring to Figs. 7 to 9, in conjunction with Fig. 2, in order to enable the balloon 100 to be inserted into and removed from the prosthesis holder 90, in a specific example, the coupling holder further includes a push shaft 110 extending axially with respect to the prosthesis holder 90 (preferably, an axis of the push shaft 110 coincides with the axis H). The push shaft 110 is inserted in the prosthesis holder 90, and the balloon 100 is attached to the push shaft 110. In this way, the balloon 100 can be moved into and out of the prosthesis holder 90 as a result of axial movement of the push shaft 110. Specifically, in the collapsed configuration, the balloon 100 is folded onto the push shaft 110 (Fig. 8). In this configuration, the balloon 100 can be moved into the prosthesis holder 90 on the push shaft 110, then the balloon 100 is inflated and expanded (see Figs. 2 and 9) to actuate the distal ends of the prosthetic members 91 to cause them to transition to the second deflected configuration.

Further, in order to facilitate collapse and expansion of the balloon 100, the push shaft 110 is configured to be inserted through the balloon 100 so that the axis of the push shaft 110 is coincident with the axis of the balloon 100 (i.e., the push shaft 110 is inserted through the balloon 100 along a long axis of the balloon 100). The proximal end and the distal end of the balloon 100 is sealingly secured to the push shaft 110 (e.g., the proximal end and the distal end of the balloon 100 is secured to the push shaft 110 by welding), and the push shaft 110 further defines a transfer channel 111 in communication with the interior of the balloon 100. In this way, an inflation medium may be introduced into or removed from the balloon 100 through the transfer channel 111 to inflate or deflate the balloon 100. The inflation medium may be a gas or liquid which is harmless to human beings.

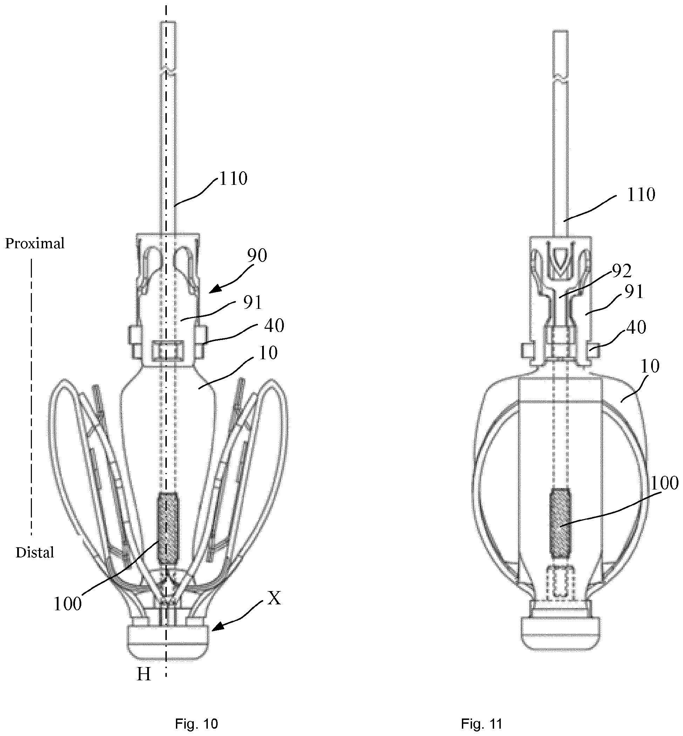

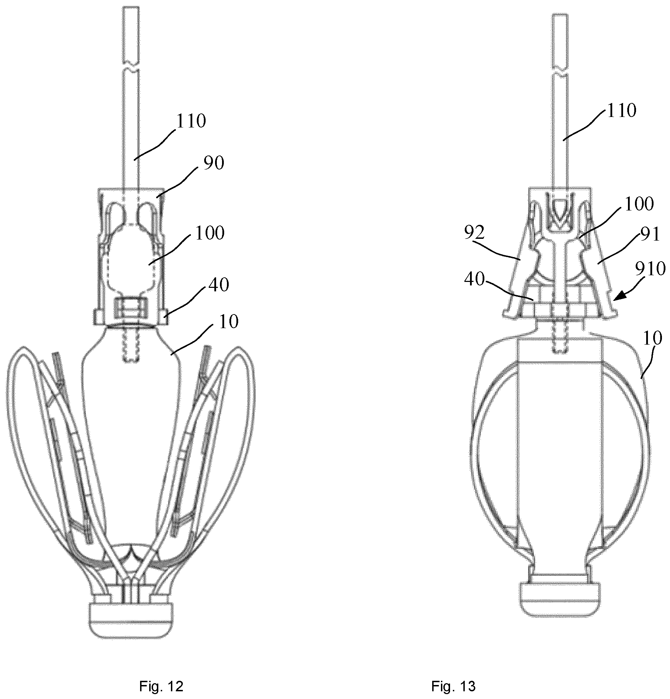

Fig. 10 shows the coupling holder assembled with a valve clip according to an embodiment of the present invention, with the prosthetic members being in the first deflected configuration, and Fig. 11 is a side view of Fig. 10. Fig. 12 shows the coupling holder separate from the valve clip according to an embodiment of the present invention, with the prosthetic members being in the second deflected configuration, and Fig. 13 is a side view of Fig. 12. Referring to Figs. 10 to 13, on the basis of the above discussed coupling holder, embodiments of the present invention also provide a valve repair system including a valve clip and the coupling holder as defined above. The valve clip includes a spacer 10, and the coupling holder can be assembled with the valve clip so that the spacer 10 is coaxial with the prosthesis holder 90, i.e., an axis of the spacer 10 is coincident with the axis H. The prosthesis holder 90 is configured to attach to a proximal end of the spacer 10 by deploying the prosthetic members 91 into the first deflected configuration. The prosthesis holder 90 is also configured to detach from the proximal end of the spacer 10 by deploying the prosthetic members 91 into the second deflected configuration. The valve clip further includes a proximal connector 40 provided at the proximal end of the spacer 10, and the spacer 10 can be assembled with the prosthesis holder 90 through the proximal connector 40. The prosthesis holder 90 can attach to the proximal connector 40 by deploying the prosthetic members 91 into the first deflected configuration. The prosthesis holder 90 can detach from the proximal connector 40 by deploying the prosthetic members 91 into the second deflected configuration. In addition, the insertion members 92 of the prosthesis holder 90 can be inserted into the proximal connector 40.

As the prosthesis holder 90 attaches to the proximal connector 40 of the valve clip by deploying the prosthetic members 91 into the first deflected configuration, the attachment of the prosthetic members 91 to the valve clip is accomplished by inward forces. This is advantageous over the prior art, in which, after a coupling holder is assembled with a valve clip, clasps of the coupling holder experience outward forces, necessitating engagement of a pushing wire with two restriction rings for preventing outward deflection of the clasps, because the inward forces enable the prosthesis holder 90 to be assembled with the valve clip without left-to-right asymmetry of the prosthetic members 91. Accordingly, the prosthetic members 91 can be preformed in the first deflected configuration and will not deflect at all after the prosthesis holder 90 is coupled to the valve clip. That is, the prosthesis holder 90 can be more stably coupled to the valve clip. Preferably, in the first deflected configuration, each prosthetic member 91 is configured so that its distal end is spaced radially with respect to the prosthesis holder 90 at a radial distance smaller than a radial distance at which its proximal end is spaced radially with respect to the prosthesis holder 90. As such, each prosthetic member 91 extends in a direction forming an angle with the axis H. This allows stronger inward forces to be exerted on the prosthetic members 91 when they attach to the proximal connector 40, which can facilitate attachment of the prosthesis holder 90 to the proximal connector 40. Moreover, as the pushing wire and restriction rings are omitted, the problem that the pushing wire may experience great resistance or make a sound when it is being advanced or retracted is circumvented. Further, according to the present invention, the balloon 100 is used to actuate the prosthetic members 91 of the prosthesis holder 90 to cause them to transition from the first deflected configuration to the second deflected configuration. Since the balloon 100 expands symmetrically, it can be ensured that equal angles between the individual prosthetic members 91 and the axis H are maintained during their transitioning from the first deflected configuration to the second deflected configuration, which can prevent difficult separation from the valve clip which may happen when one or more of the prosthetic members 91 deflect to an insufficiently large extent in the second deflected configuration.

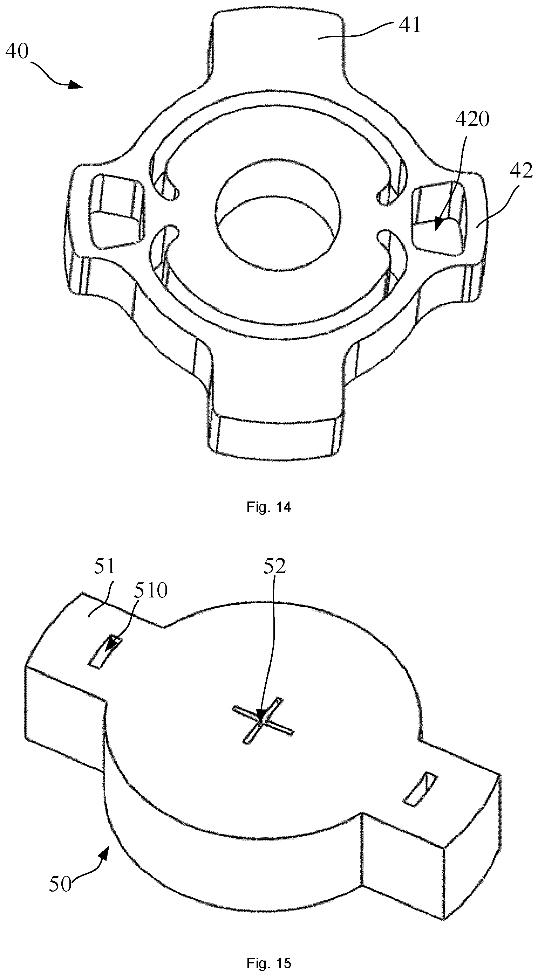

The valve clip is detachably coupled to the coupling instrument. Specifically, reference is made to Fig. 14, Fig. 14 is a schematic illustration of the proximal connector according to an embodiment of the present invention. The proximal connector 40 has at least two first extensions 41 (in the example shown in Fig. 14, there are two symmetrical first extensions 41), which are raised outwardly (away from the axis H) radially with respect to the spacer 10. The proximal connector 40 also has at least two second extensions 42 which are outwardly raised radially with respect to the spacer 10. Each second extension 42 defines a through insertion slot 420. During assembly, the balloon 100 is collapsed on the push shaft 110, the balloon 100 is inserted thereon through the prosthesis holder 90 and the proximal connector 40 into the spacer 10, and a mechanical tool is then used to outwardly move the distal ends of the prosthetic members 91 to cause them to transition to the second deflected configuration. After that, the insertion members 92 are inserted into the insertion slots 420 of the proximal connector 40, and the mechanical tool is gradually withdrawn to allow the first extensions 41 to snap-engage into the prosthetic slots 910 of the prosthetic members 91. In this way, the coupling instrument can be assembled with the valve clip by means of the proximal connector 40. After the coupling instrument is assembled with the valve clip, the push shaft 110 may be manipulated to actuate the coupling instrument and valve clip, as a whole. Specifically, the valve clip further includes a connector assembly X disposed at a distal end of the spacer 10. The connector assembly X can be detachably (i.e., threadedly) connected to the push shaft 110. After being secured to the connector assembly X, the push shaft 110 can be manipulated to advance the valve clip into, or retract it from, a patient's body. During release, the push shaft 110 is disconnected from the connector assembly X and pulled to remove the collapsed balloon 100 thereon from the spacer 10 and retract it back into the prosthesis holder 90. The balloon 100 is then inflated to expand to deflect the distal ends of the prosthetic members 91 to cause them to transition to the second deflected configuration. As a result, the first extensions 41 are separated from the prosthetic slots 910 of the prosthetic members 91. Subsequently, the insertion members 92 are removed from the insertion slots 420, achieving release of the coupling instrument from the valve clip.

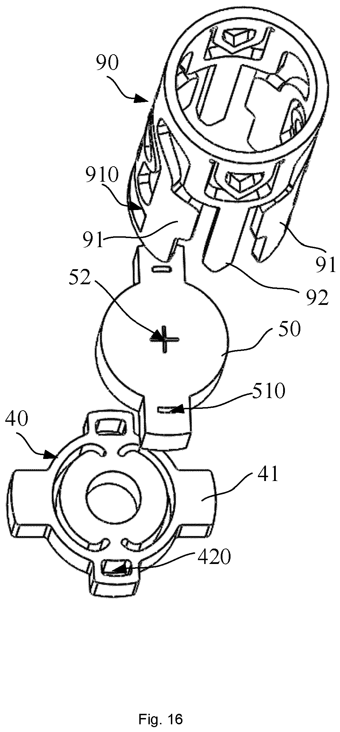

Fig. 15 is a schematic illustration of a sealing gasket according to an embodiment of the present invention. Fig. 16 is an exploded view of the prosthesis holder, the sealing gasket and the proximal connector according to an embodiment of the present invention. Preferably, referring to Figs. 15 and 16, the valve clip further includes a sealing gasket 50 connected to the proximal connector 40. The sealing gasket 50 has at least two third extensions 51 extending outwardly radially with respect to the spacer 10. Each third extension 51 defines a through first slot 510, and the sealing gasket 50 defines a second slot 52 at its center. The insertion members 92 of the prosthesis holder 90 can be successively inserted through the first slots 510 and the insertion slots 420 of the proximal connector 40, and the push shaft 110 can be inserted successively through the prosthesis holder 90, the second slot 52 of the sealing gasket 50 and the proximal connector 40 into the spacer 10 and secured to the connector assembly X of the spacer 10. When the valve repair system is introduced into a patient's body, the sealing gasket 50 can prevent ingress of blood into the valve clip or coupling instrument, making the surgical procedure less risky. The sealing gasket 50 may be made of silicone, i.e., it may be a silicone gasket. The first slots 510 on the sealing gasket 50 can facilitate separation of the coupling instrument from the valve clip. Specifically, the first slots 510 can reduce friction between the insertion members 92 and the sealing gasket 50 and thereby facilitate removal of the insertion members 92 successively from the proximal connector 40 and the sealing gasket 50. The second slot 52 can facilitate not only advancement or retraction of the push shaft 110 but also sealing of the sealing gasket 50 with the push shaft 110, which can prevent ingress of blood into the valve clip during operation of the push shaft 110. The insertion members 92 of the prosthesis holder 90 are generally thin sheets, and the first slots 510 and the second slot 52 are preferably all slit cutouts. Such shapes allow the sealing gasket 50 to fit onto the exterior of the insertion members 92 across the entire extent of the efirst slots 510 and the second slot 52. Compared with conventional sealing techniques based on only slots, the sealing gasket 50 can provide better sealing performance.

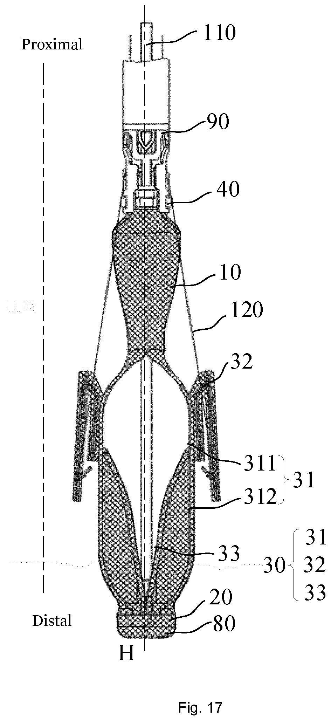

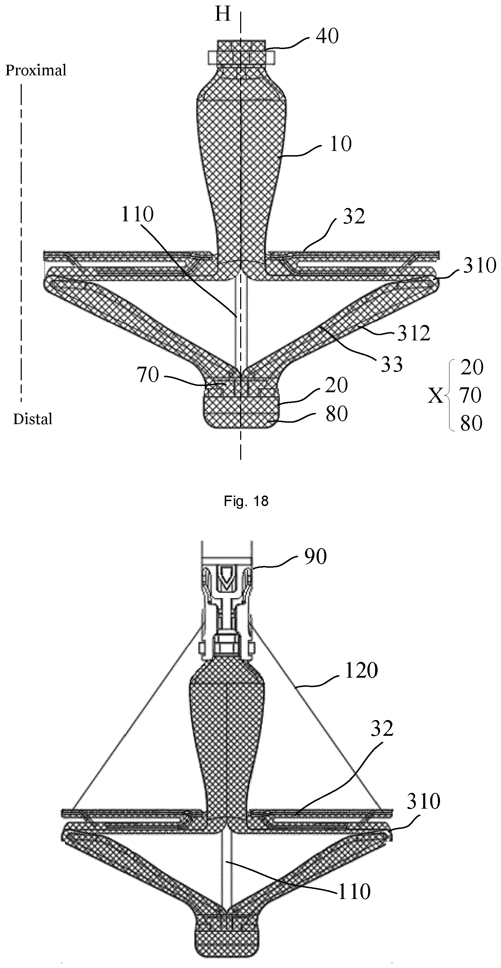

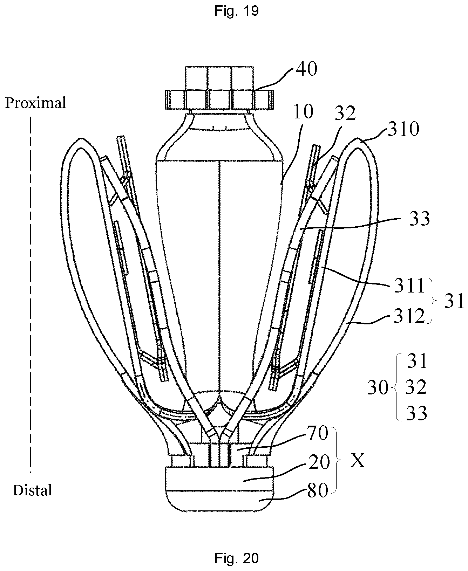

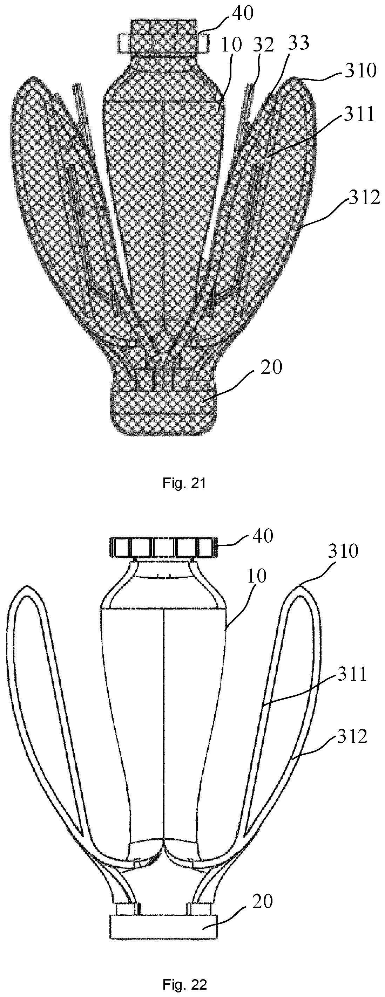

The valve clip according to embodiments of the present invention is described in detail below with reference to Figs. 17 to 22. Fig. 17 is a schematic illustration of the valve clip according to an embodiment of the present invention, in which spacing arms are in a first configuration. Figs. 18 and 19 are schematic illustrations of the valve clip according to an embodiment of the present invention, in which the spacing arms are in a second configuration. Figs. 20 and 21 are schematic illustrations of the valve clip according to an embodiment of the present invention, in which the spacing arms are in a third configuration. Fig. 22 schematically illustrates the spacing arms in the third configuration and the spacer according to an embodiment of the present invention. Notably, the balloon 100 of the coupling instrument is omitted in Figs. 17 to 22.

As shown in Figs. 17 to 22, the valve clip includes the spacer 10, the connector assembly X, two repair assemblies 30 and the above-discussed proximal connector 40 and sealing gasket 50. The spacer 10 is generally shaped like an ellipsoid and braided of metal wires. For example, the spacer 10 may be braided of nickel-titanium alloy wires and then shape-set using a heat setting technique. The connector assembly X is arranged movably relative to the spacer 10 along an axis of the spacer 10, after the valve clip is assembled with the coupling instrument, the connector assembly X can move along the axis H. Specifically, the push shaft 110 can be inserted through the spacer 10 and secured to the connector assembly X, allowing the connector assembly X to be moved by manipulating the push shaft 110. The two repair assemblies 30 are centrosymmetric about the center of the spacer 10 (i.e., symmetrical about the axis H). In other words, the two repair assemblies 30 are diametrically symmetrical with respect to the spacer 10.

Each repair assembly 30 includes a spacing arm 31, a clasp 32 and a compression ring 33. The spacing arm 31 includes a capture section 311 and a support section 312, both extending along the axis of the spacer 10. As viewed in the orientation of Fig. 17, the spacer 10, the capture section 311 and the support section 312 are connected in order from proximal to distal. As shown in Fig. 17, the capture section 311 and the support section 312 both extend along the axis of the spacer 10 (parallel to the axis H) and are joined to each other. A distal end of the capture section 311 is connected to a proximal end of the support section 312 to form a spacing portion 310. The connection between the capture section 311 and the support section 312 is defined as the spacing portion 310. The spacing arm 31 is an elastic component generally shaped like a band or plate. That is, the spacing arm 31 is an elastic component, which has elasticity and is made of an elastic material. In other words, the spacing arm 31 can deform, i.e., change the shapes of the capture section 311 and the support section 312, under the action of external forces. For example, the capture section 311 and the support section 312 can overall assume a folded configuration. The distal end of the support section 312 is connected to the connector assembly X. Notably, the spacing arm 31 may be braided of metal wires (e.g., nickel-titanium alloy wires). The spacing arm 31 may be connected to the connector assembly X by laser welding. The spacing arm 31 may be integrally formed with the spacer 10. Alternatively, they may be separately fabricated and then connected to each other. The clasp 32 is disposed on the capture section 311 axially with respect to the spacer 10 and adapted to clamp a predetermined target. As used herein, the term "predetermined target" generally refers to a leaflet of the mitral or tricuspid valve. Of course, it may also refer to a leaflet of another native valve such as the aortic or pulmonary valve. The proximal end of the compression ring 33 is connected to the spacing portion 310 and the distal end of the compression ring 33 is connected to the connector assembly X. The compression ring 33 is adapted to exert a force on the spacing arm 31. Notably, the compression ring 33 and the clasp 32 are each generally formed by laser-cutting a nickel-titanium alloy article and then shape-setting the cut article using a heat setting process. Thus, the compression ring 33 and the clasp 32 are both elastic and able to elastically deform.

The valve clip is configured so that the spacing arms 31 can be urged by the compression rings 33 to move along with the connector assembly X (e.g., from proximal to distal, or from distal to proximal) to transition between different configurations, typically between the first and second configurations and between the second and third configurations. Here, the second configuration should be construed as any intermediate configuration between the first and third configurations. Notably, when the coupling instrument is assembled with the valve clip, the center axis of the spacer 10 is coincident with the axis H. Referring to Fig. 17, when the spacing arm 31 is in the first configuration, the capture section 311 and the support section 312 are substantially collinear and brought close to the center axis of the spacer 10. It should be understood that, here, the term "colinear" should be interpreted in a broad sense. That is, it means the sections are essentially collinear. Additionally, the term can be interpreted as meaning that there is no bend at the spacing portion 310. In the first configuration of the spacing arms 31, when the spacing portions 310 are projected inwardly radially with respect to the spacer 10 onto the center axis thereof, the projections are located outside of the extent of the spacer 10. More precisely, axial positions of the spacing portions 310 on the axis of the spacer 10 are external to the distal end of the spacer 10. The two spacing arms 31 define an overall shape, which, when viewed from the front, substantially appears like an ellipse (see Fig. 17). Referring to Figs. 18 and 19, when the spacing arms 31 are in the second configuration, the spacing arms 31 deflect outwardly radially with respect to the spacer 10, and axial positions of the spacing portions 310 on the axis of the spacer 10 are also external to the distal end of the spacer 10. That is, when the spacing portions 310 are projected radially with respect to the spacer 10 onto the center axis thereof, the projections are located outside of the extent of the spacer 10. The spacing arms 31 transition from the first configuration to the second configuration as a result of gradual outward movement of their ellipsoidal middle portions around the spacing portions 310. In particular, referring to Figs. 18 and 19, in the second configuration of the spacing arms 31, when the connector assembly X reaches a limit for its movement from distal to proximal (for ease of description, the configuration at this time is referred to here as a second perpendicular configuration), the capture sections 311 become perpendicular to the center axis of the spacer 10, and the spacing arms 31 of the repair assemblies 30 overall define a shape like an isosceles triangle. As would be appreciated, here, the perpendicularity of the capture sections 311 to the center axis of the spacer 10 should be interpreted in a broad sense. For example, the capture sections 311 can be considered perpendicular to the center axis of the spacer 10 if angles that they form with the axis lie in the range [90°-5°, 90°+5°]. As shown in Figs. 20 and 21, when the spacing arms 31 are in the third configuration, the spacing arms 31 deflect outwardly radially with respect to the spacer 10, and when the spacing portions 310 are projected inwardly radially with respect to the spacer 10, the projections are located on the spacer 10, i.e., axial positions of the spacing portions 310 on the axis of the spacer 10 are within the extent of the spacer 10. Moreover, the capture sections 311 and the support sections 312 assume a folded configuration in which they are brought close to the spacer 10. That is, the capture sections 311 and the support sections 312 are urged by the compression rings 33 toward the spacer 10. Notably, to reduce injury that the valve clip may cause to human tissue, the exterior of the valve clip is usually covered with at least one coating film typically made of a polymer material for medical use. In Figs. 17, 18, 19 and 20, the valve clip is depicted as being covered with a coating film shown cross-hatched.

When used in practical edge-to-edge valve repair surgery, the valve clip can be implanted into a patient's ventricle with the spacing arms 31 being deployed in the first configuration. The spacing arms 31 are then caused to transition to the third configuration and placed along with the spacer 10 between the leaflets of the mitral or tricuspid valve. Additionally, the spacing arms 31 are caused to transition to the second configuration (preferably, the second perpendicular configuration), followed by capturing and clamping the leaflets by the clasps 32 on the capture sections 311. After the clasps 32 of both repair assemblies 30 have desirably fastened the respective leaflets, the spacing arms 31 are finally caused to transition to the third configuration, achieving edge-to-edge repair of the mitral or tricuspid valve. Notably, the clasps 32 are configured to capture and clamp leaflets when the spacing arms 31 are in the second perpendicular configuration. However, in practice, they usually achieve this when the spacing arms 31 are in an intermediate configuration between the second perpendicular configuration and the third configuration. As would be appreciated, the clasps 32 capture the leaflets as soon as the spacing arms 31 transition to the third configuration, when projections of the spacing portions 310 on the center axis of the spacer 10 are located at the distal end of the spacer 10. The spatial form and volume of the spacer 10 can prevent the leaflets from being ruptured during fastening and repair. According to embodiments of the present invention, through configuring the spacing arms 31 so that they can transition between the different configurations under the joint action of the compression rings 33 and the connector assembly X and through providing the clasps 32 on the capture sections 311 so that they can change their positions as the spacing arms 31 are transitioning between the configurations, a significantly increased success rate can be obtained in implantation of the clasps 32, capture and fastening of leaflets and edge-to-edge value repair, and implantation failure can be minimized. This not only enables safer, more reliable and more stable implantation, but also allows for easier surgical operation, a shorter surgical time, increased surgical safety and reliability, reduced surgical risks that may be caused by the components of the system, and effective prevention of mitral or tricuspid valve regurgitation. Further, when in the third configuration, the spacing arms 31 are urged by the compression rings 33 toward the spacer 10. This enables even better repair of the mitral or tricuspid valve in terms of morphology and reduces the risk of reoccurrence of mitral or tricuspid valve regurgitation. Furthermore, the coupling instrument can be coupled to the valve clip so that the prosthetic members 91 of the coupling instrument are symmetrical about the aforementioned axis without one or more of them deflecting to an insufficiently large extent. As such, the coupling instrument can be more stably coupled to the proximal connector 40, additionally facilitating the surgeon's operation and reducing surgical risks.

Generally, operation of the valve clip is controlled by an external delivery system. Such control may include, but is not limited to, implanting the valve clip system into a patient's body and advancing it into the patient's ventricle; causing the spacing arms 31 to transition between the three configurations; controlling operation of the clasps 32; and after the valve clip completes mitral or tricuspid valve repair, releasing the delivery system from the valve clip and withdrawing it from the patient's body. Specifically, such a delivery system is connected to the valve clip through the coupling instrument and may include control wires 120. The push shaft 110 of the coupling instrument may be manipulated to actuate the connector assembly X and advance the valve clip into a patient's body. The control wires 120 may be manipulated to control operation of the clasps 32 (for clamping or releasing leaflets). Referring to Figs. 18, 20, 21 and 22, the valve clip is coupled to the prosthesis holder 90 through the proximal connector 40 on the spacer 10, and the push shaft is inserted successively through the coupling instrument, the sealing gasket 50, the proximal connector 40 and the spacer 10 and secured to the connector assembly X. Moving the push shaft 110 forth and back along the axis of the spacer 10 can cause the valve clip to transition between the first, second and third configurations.

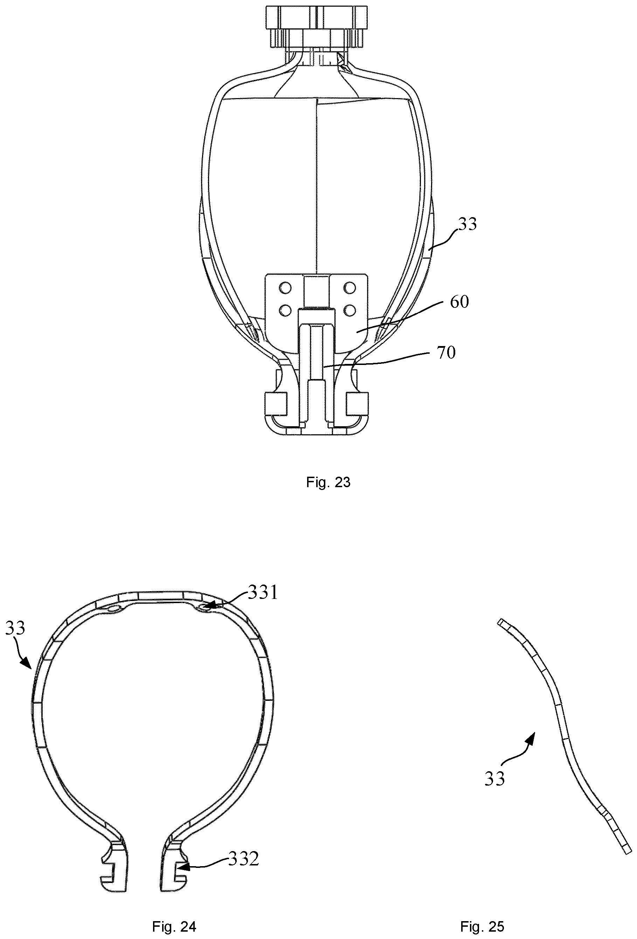

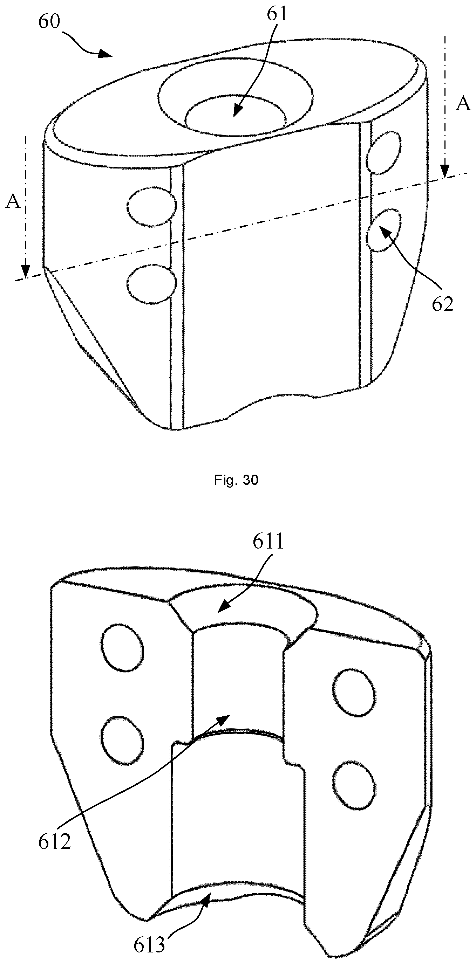

Fig. 23 is a cross-sectional view of the valve clip taken along the axis of the spacer, according to an embodiment of the present invention, in which the spacing arms are in the third configuration. Fig. 30 is a schematic illustration of a support body according to an embodiment of the present invention. Referring to Figs. 23 and 30, the valve clip further includes a support body 60, the support body 60 is disposed within the spacer 10 and affixed around the distal end of the spacer 10. The support body 60 defines a first guide bore 61 extending therethrough along the axis of the spacer 10, the first guide bore 61 is configured for passage of the push shaft 110 therethrough. Generally, the push shaft 110 is inserted successively through the sealing gasket 50, the proximal connector 40, the spacer 10 and the support body 60 and eventually secured to the connector assembly X. In particular, the support body 60 may be affixed to the spacer 10 by a suture passed through the support holes 62 in the support body 60.

Fig. 31 is a cross-sectional view of Fig. 30 taken along line A-A. Referring to Fig. 31, the first guide bore 61 preferably includes a first bore section 611, a second bore section 612 and a third bore section 613, which are sequentially joined in order from proximal to distal. Both the first bore section 611 and the third bore section 613 have a greater radial dimension than the second bore section 612. The first bore section 611 is tapered from proximal to distal while the third bore section 613 is flared from proximal to distal. Notably, here, the radial dimension of the first guide bore 61 refers to its maximum radial dimension, and when the first guide bore 61 is circular, to its maximum diameter. With this arrangement, the first bore section 611 resembles an inverted frustoconical bore, while the third bore section 613 resembles a non-inverted frustoconical bore. In practice, after the valve clip is delivered into a patient's ventricle, it is steered to in between the leaflets of the mitral or tricuspid valve. The steering of the valve clip can be accomplished by manipulating the push shaft 110. According to the present invention, the push shaft 110 can move with more freedom within the first guide bore 61 that includes the first bore section 611, the second bore section 612 and the third bore section 613, facilitating guidance of the valve clip.

Referring to Figs. 18 and 20, notably, according to embodiments of the present invention, the connector assembly X includes a guidewire connector 70, a distal connector 20 and a cap connector 80 in order from proximal to distal. The guidewire connector 70 is snap-fastened to the distal connector 20, and the cap connector 80 is threadedly secured to the guidewire connector 70.

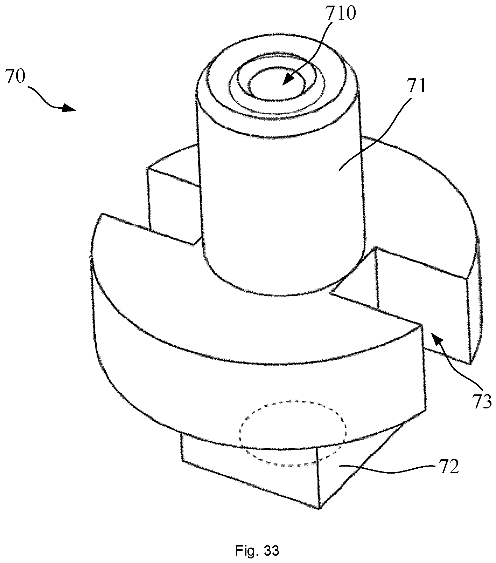

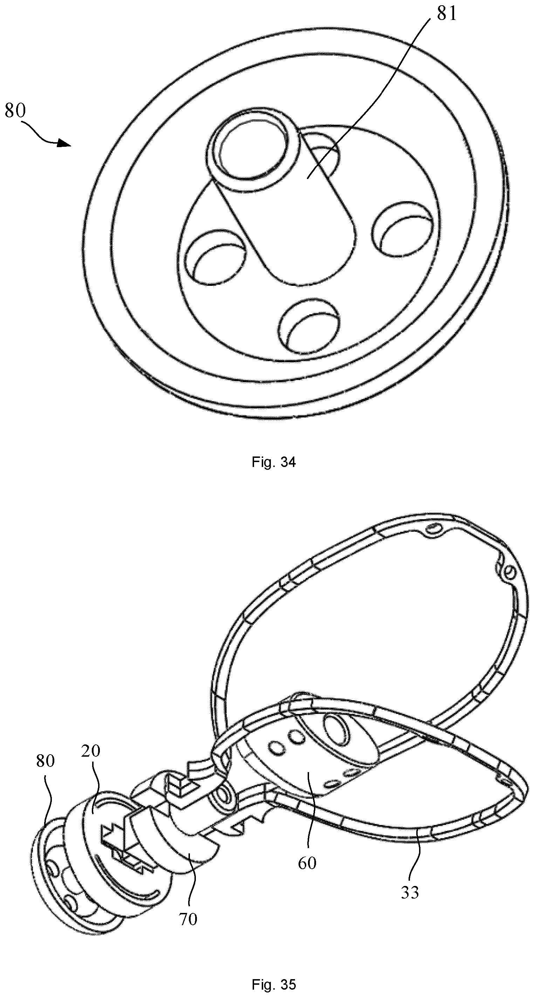

Fig. 33 is a schematic illustration of the guidewire connector according to an embodiment of the present invention. Fig. 34 is a schematic illustration of the cap connector according to an embodiment of the present invention. Referring to Figs. 33 and 34, the guidewire connector 70 defines a second guide bore 710, an axis of the second guide bore 710 is parallel to the axis of the spacer 10. The cap connector 80 has a fifth extension 81 projecting towards the support body 60. The fifth extension 81 is adapted to be threadedly engaged in the second guide bore 710, thereby threadedly securing the guidewire connector 70 to the cap connector 80. The second guide bore 710 may be a through bore or a blind bore. In the latter case, the proximal end of the second guide bore 710 is closed. The distal end of the push shaft 110 is connected to the guidewire connector 70, for example, the push shaft 110 is secured over a proximal section of the second guide bore 710. For example, the distal end portion of the push shaft 110 may be provided with an external thread 112 (see Figs. 2 and 7), the external thread 112 can threadedly engage the proximal section of the second guide bore 710.

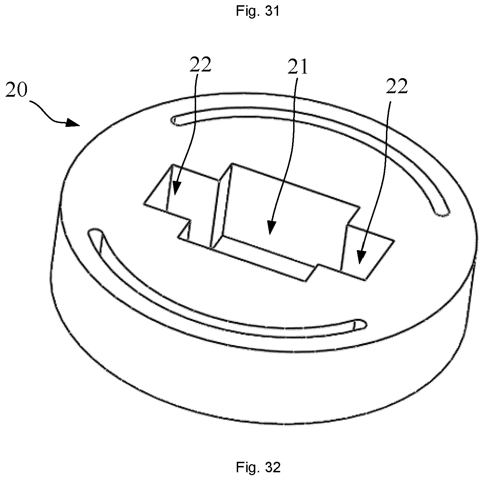

Fig. 32 is a schematic illustration of the distal connector according to an embodiment of the present invention. Referring to Fig. 32, in conjunction with Fig. 33, the guidewire connector 70 has a sixth extension 72 projecting away from the spacer 10, and the distal connector 20 defines a connector receptacle 21, the connector receptacle 21 is recessed from its proximal to distal end and complementary to the sixth extension 72. The sixth extension 72 can be snapped into the connector receptacle 21, thereby snap-fastening the distal connector 20 to the guidewire connector 70. The guidewire connector 70 may have a fourth extension 71 which projects towards the spacer 10 and defines part of the second guide bore 710, and the sixth extension 72 may define the rest of the second guide bore 710. Further, the fourth extension 71 may move into or out of the first guide bore 61 of the support body 60 as a result of corresponding movement of the connector assembly X.

The present invention is not limited to any particular method of connecting the compression rings 33 to the spacing arms 31 and the connector assembly X. Reference is now made to Figs. 24 and 25. Fig. 24 is a schematic illustration of one compression ring according to an embodiment of the present invention, and Fig. 25 is a side view of Fig. 24. In an exemplary embodiment, each compression ring 33 is provided at its proximal end with at least two through compression holes 331, through which a suture can be passed to attach the compression ring 33 to a respective one of the spacing portions 310. In another exemplary embodiment, referring to Figs. 24 and 32, the compression rings 33 are configured for engagement with the distal connector 20 in the connector assembly X. The distal connector 20 defines first engagement recesses 22, the first engagement recesses 22 are outwardly recessed radially with respect to the spacer 10, and the distal end of each compression ring 33 defines mating second engagement recesses 332 which are inwardly recessed radially with respect to the spacer 10. The compression rings 33 can be engaged with the distal connector 20 by engaging the first engagement recesses 22 with the second engagement recesses 332. Reference is additionally made to Fig. 35, Fig. 35 shows the compression rings 33 being assembled with the distal connector 20 according to an embodiment of the present invention. In order to assemble the compression rings 33 with the distal connector 20, the distal ends of the compression ring 33 may be passed through notches 73 in the guidewire connector 70 and then engaged in the first engagement recesses 22.

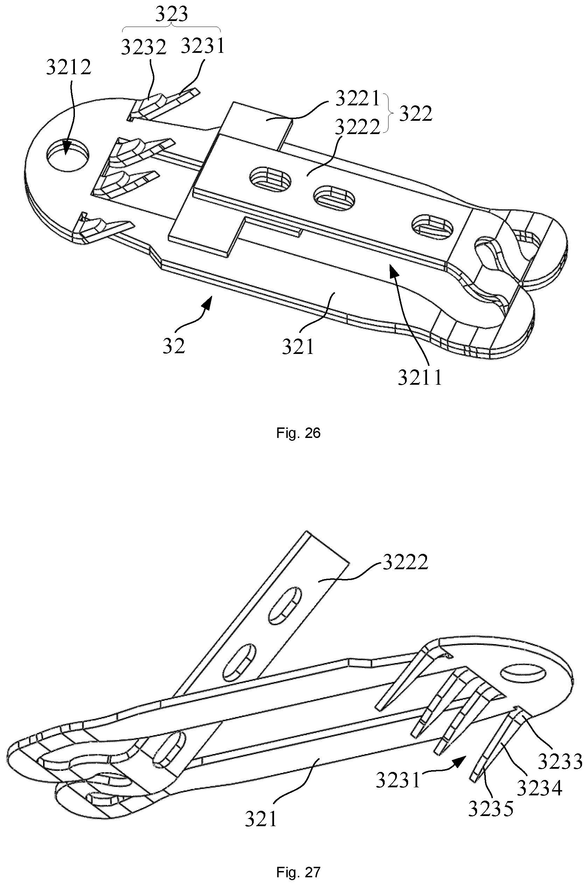

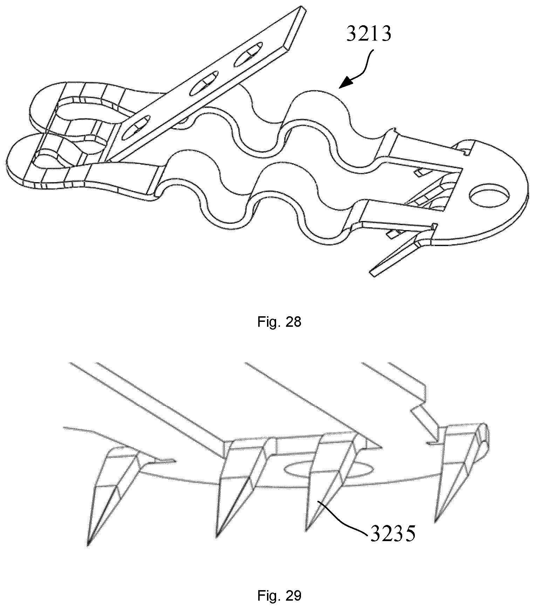

Figs. 26 and 27 are schematic illustrations of one clasp according to an embodiment of the present invention. Fig. 28 is another schematic illustration of the clip according to an embodiment of the present invention. Referring to Figs. 26 to 28, each clasp 32 includes clasping sheet 321 and a support sheet 322. The support sheet 322 is affixed to a respective one of the capture sections 311. The support sheet 322 is configured to fit onto the capture section 311. Preferably, the support sheet 322 extends parallel to the capture section 311. The clasping sheet 321 is elastically joined at one end to the support sheet 322 so as to be elastically biased toward the support sheet 322. The clasps 32 can be located at different positions in the different configurations of the spacing arms 31. The control wires 120 may be manipulated to actuate the clasps 32 to capture and clamp leaflets when the spacing arms 31 are in the second configuration, preferably in the second perpendicular configuration, or as soon as the spacing arms 31 transition to the third configuration. Specifically, as noted above, the clasping sheet 321 is elastically joined to the support sheet 322 so as to be elastically biased toward the support sheet 322, and the support sheet 322 is affixed to the respective capture section 311. A substantially proximal external force can be applied to the clasping sheet 321 to deflect the clasping sheet 321 away from the support sheet 322, bringing the clasp into an open configuration in which the clasp substantially resembles a ">"-like shape. It is suitable for the clasp in this configuration to capture a leaflet. When the external force is removed, the clasping sheet 321 will elastically deflect back to fasten the leaflet between it and the support sheet 322. It will be appreciated that the magnitude of the external force can be changed to adjust the opening between the clasping sheet 321 and the support sheet 322. That is, the angle of the opening of the ">"-like shape can be controlled by controlling the magnitude of the external force. Here, the external force is typically applied by a respective one of the control wires 120 in the delivery system.

In greater detail, the clasping sheet 321 defines an elongate through slot 3211, a lengthwise direction of the elongate slot 3211 is parallel to the capture section 311. The support sheet 322 includes a transverse part 3221 and a longitudinal part 3222, which together define a shape like the letter "T". The longitudinal part 3222 extends in the lengthwise direction of the elongate slot 3211 within the extent of the elongate slot 3211, and the transverse part 3221 extends in a widthwise direction of the elongate slot 3211 outside of the extent of the elongate slot 3211. The transverse part 3221 is adapted to abut against the clasping sheet 321 to prevent the longitudinal part 3222 from elastically deflecting into the elongate slot 3211.

Fig. 29 is a schematic illustration of barbs on one clip according to an embodiment of the present invention. Referring to Fig. 29, the clasping sheet 321 is preferably provided with barbs 323, the barbs 323 including long barbs 3231 and short barbs 3232. The long barbs 3231 are longer than the short barbs 3232. Each long barb 3231 includes a fixed portion 3233, an intermediate portion 3234 and a tapered portion 3235, which are joined in this order. The fixed portion 3233 is joined to the clasping sheet 321. The short barbs 3232 are provided to support and stabilize the long barbs 3231. The tapered portions 3235 of the long barbs 3231 can pierce and anchor into leaflet tissue, allowing the clasp 32 to capture and clamp the leaflet more stably and reliably. In practice, the long barbs 3231 may be made as long as permissible to allow them to desirably reach a greater depth in leaflet tissue.

Referring to Fig. 29, the present invention is not limited to any particular shape of the tapered portion 3235, and examples of the shape may include, but are not limited to, cones and pyramids (e.g., quadrangular pyramids, as shown in Fig. 29). Conventionally, barbs 323 with tapered portions 3235 in the shape of flat triangular sheets are used. In contrast, the three-dimensional conical or pyramidal shape of the tapered portions 3235 of the barbs 323 according to the present invention allows the long barbs 3231 of the barbs 323 to more easily pierce and anchor into leaflet tissue and to experience even circumferential stress within the leaflet tissue, thereby reducing damage caused to the leaflet and allowing for more secure anchoring.

Referring to Fig. 28, the clasping sheet 321 is preferably telescopic in its own direction of extension (i.e., the direction of extension of the capture section 311). Here, by "telescopic", it is intended to mean that clasping sheet 321 is extendable and retractable, or elastic in its own direction of extension. In this way, when the respective control wire 120 is manipulated to actuate the clasping sheet 321 toward an open configuration with respect to the support sheet 322, the clasping sheet 321 can be adaptively stretched to accommodate patients with severe leaflet prolapse or a large gap between the anterior and posterior leaflets. With this arrangement, when a leaflet is clamped between the clasping sheet 321 and the support sheet 322, the long barbs 3231 will pierce and anchor into the leaflet tissue. Moreover, after an external force exerted by the control wire 120 is removed, the clasping sheet 321 will gradually elastically deflect back to pull the leaflet. This enables better leaflet fastening and repair. The present invention is not limited to any particular method of making the clasping sheet 321 elastic or telescopic. In an exemplary embodiment, with continued reference to Fig. 28, the clasping sheet 321 is made of an elastic material, the clasping sheet 321 includes a number of telescopic sections 3213 joined along its own direction of extension. The telescopic sections 3213 together define a shape substantially like waves (including but not limited to smooth waves and serrated waves). The telescopic sections 3213 can be stretched under the action of an external force and will restore the original shape after the external force is removed.

According to embodiments of the present invention, the valve repair system includes the coupling instrument and the valve clip, and the coupling instrument couples the valve clip to the delivery system including the control wires 120. The push shaft 110 of the coupling instrument is adapted to actuate the connector assembly X along the axis of the spacer 10, thereby causing the valve clip to transition between the first and second configurations and between the second and third configurations. The control wires 120 are tied to the clasps 32 and can be adapted to control operation of the clasps 32. That is, the clasps 32 can be manipulated, for example, to capture and clamp leaflets. Further, when the clasps 32 fail to clamp leaflets in a desired way, they may be again manipulated to make another attempt to capture and clamp the leaflets.

In an exemplary embodiment, referring to Figs. 17, 19 and 26, the clasping sheet 321 defines a control slot 3212, the clasping sheet 321 is tied to the respective control wire 120 via the control slot 3212. The two control wires 120 are tied to the respective clasping sheets 321. During surgery, after spacing arms 31 are deployed into the second configuration, preferably into the second perpendicular configuration, under the joint action of the push shaft 110, the connector assembly X and the compression rings 33, as soon as the spacing arms 31 transition to the third configuration, the control wires 120 can be manipulated to deflect the clasping sheets 321 away from the support sheets 322 to allow leaflets to be captured and clamped therebetween. In particular, the clamping of the leaflets by the clasps 32 can be observed using an external ultrasonography system. If satisfactory clamping is not attained, the control wires 120 may be again manipulated to make another attempt to capture and clamp the leaflets. Notably, the two control wires 120 can be manipulated without mutual interference. They may be simultaneously manipulated to operate the respective clasps 32 at the same time. Alternatively, at any time, only one of them may be manipulated to operate the respective clasps 32. Thus, the clasping sheets 321 can be individually and separately opened to capture leaflets to accommodate applications requiring operation of only a single clasp 32 during surgery.

It will be appreciated that when the clasps 32 of the valve clip succeed in clamping and fastening a mitral or tricuspid valve in a desirable way, the spacing arms 31 of the valve clip are in the third configuration, with the leaflets of the mitral or tricuspid valve being clamped by the clasps 32. The push shaft 110 and the compression rings 33 are then operated to actuate the spacing arms 31 inwardly toward the spacer 10. At this time, it can be considered that the surgical procedure has been satisfactorily performed. Subsequently, the push shaft 110 is manipulated to be separated from the connector assembly X and retracted back into the prosthesis holder 90. After that, the balloon 100 is inflated to expand to actuate the prosthetic members 91 into the second deflected configuration, in which the first extensions 41 are separated from the prosthetic members 91. Afterwards, the insertion members 92 are removed from the insertion slots 420, separating the coupling instrument from the valve clip. Both the coupling instrument and the control wires 120 are then withdrawn from the patient's body.

In summary, the present invention provides a valve repair system and a coupling instrument thereof. The coupling instrument includes a prosthesis holder and a balloon. The prosthesis holder includes at least two prosthetic members, which are arranged circumferentially around an axis of the prosthesis holder and proximally fixed in position radially with respect to the prosthesis holder. The balloon can be inflated to expand to actuate distal ends of the prosthetic members to cause them to transition from a first deflected configuration into a second deflected configuration. In the first deflected configuration, each prosthetic member is configured so that its distal end is spaced radially with respect to the prosthesis holder at a radial distance smaller than or equal to a radial distance at which its proximal end is spaced radially with respect to the prosthesis holder. In the second deflected configuration, each prosthetic member is configured so that its distal end is spaced radially with respect to the prosthesis holder at a radial distance greater than a radial distance at which its proximal end is spaced radially with respect to the prosthesis holder. As the prosthesis holder is assembled with the valve clip by deploying the prosthetic members into the first deflected configuration, the attachment of the prosthetic members to the valve clip is accomplished by inward forces. This is advantageous over the prior art, in which, after a coupling instrument is assembled with a valve clip, clasps of the coupling instrument experience outward forces, necessitating engagement of a pushing wire with two restriction rings for preventing outward deflection of the clasps, because the inward forces enable the prosthesis holder to be assembled with the valve clip without left-to-right asymmetry of the prosthetic members. Accordingly, the prosthetic members can be preformed in the first deflected configuration and will not deflect at all after the prosthesis holder is coupled to the valve clip. That is, the prosthesis holder can be more stably coupled to the valve clip. Moreover, as the pushing wire and restriction rings are omitted, the problem that the pushing wire may experience great resistance or make a sound when it is being advanced or retracted is circumvented. Further, according to the present invention, the balloon is used to actuate the prosthetic members of the prosthesis holder to cause them to transition from the first deflected configuration to the second deflected configuration. Since the balloon expands symmetrically, it can be ensured that equal angles between the individual prosthetic members and an axis of the prosthesis holder are maintained during their transitioning from the first deflected configuration to the second deflected configuration, which can prevent difficult separation from the valve clip which may happen when one or more of the prosthetic members deflect to an insufficiently large extent in the second deflected configuration.

The description presented above is merely that of a few preferred embodiments of the present invention and is not intended to limit the scope thereof in any sense. Any and all changes and modifications made by those of ordinary skill in the art based on the above teachings fall within the scope of the invention.

## Claims

1. A coupling instrument used in a valve repair system, comprising a prosthesis holder and a balloon,
the prosthesis holder comprising at least two prosthetic members arranged circumferentially around an axis of the prosthesis holder, proximal ends of the prosthetic members fixed in position radially with respect to the prosthesis holder, the balloon inflatable to expand to actuate distal ends of the prosthetic members to cause the prosthetic members to transition from a first deflected configuration into a second deflected configuration,
each prosthetic member configured in the first deflected configuration so that a distal end of the prosthetic member is spaced radially with respect to the prosthesis holder at a radial distance smaller than or equal to a radial distance at which a proximal end of the prosthetic member is spaced radially with respect to the prosthesis holder, each prosthetic member configured in the second deflected configuration so that the distal end of the prosthetic member is spaced radially with respect to the prosthesis holder at a radial distance greater than a radial distance at which the proximal end of the prosthetic member is spaced radially with respect to the prosthesis holder.

2. The coupling instrument according to claim 1, wherein the balloon is disposed coaxial with the prosthesis holder.

3. The coupling instrument according to claim 1, wherein when the prosthetic members are in the first deflected configuration, the distal end of each prosthetic member is spaced at an equal radial distance radially with respect to the prosthesis holder.

4. The coupling instrument according to claim 1, comprising a push shaft extending along the axis of the prosthesis holder, wherein the push shaft is inserted within the prosthesis holder, the push shaft attached to the balloon and adapted to move axially to bring the balloon into, or remove the balloon from, the prosthesis holder.

5. The coupling instrument according to claim 4, wherein the push shaft is inserted through the balloon, wherein a proximal end and a distal end of the balloon is sealingly attached to the push shaft, and wherein the push shaft defines a transfer channel in communication with an interior of the balloon.

6. The coupling instrument according to claim 4, wherein a distal end of the push shaft is provided with an external thread.

7. The coupling instrument according to claim 1, wherein the distal end of each prosthetic member defines a prosthetic slot, the prosthetic slot extending through the specific prosthetic member radially with respect to the prosthesis holder.

8. The coupling instrument according to claim 1, wherein the at least two prosthetic members are symmetric about a center of the prosthesis holder.

9. The coupling instrument according to claim 1, wherein the prosthetic members are made of a shape memory alloy, an initial configuration of the prosthetic members is configured as the first deflected configuration.

10. The coupling instrument according to claim 1, wherein the prosthesis holder further comprises at least two insertion members, which are arranged circumferentially around and extend along the axis of the prosthesis holder.

11. A valve repair system, comprising a valve clip and the coupling instrument of any one of claims 1 to 10, the valve clip detachably coupled to the coupling instrument,
the valve clip comprising:
a spacer disposed coaxial with the prosthesis holder;
a connector assembly which is movable relative to the spacer along an axis of the spacer; and
two repair assemblies arranged symmetrically about a center of the spacer, each repair assembly comprising:
an elastic spacing arm comprising a capture section and a support section, both extending along the axis of the spacer, wherein the spacer, the capture section and the support section are joined in order from proximal to distal, wherein a distal end of the capture section is joined to a proximal end of the support section at a joint defining a spacing portion, and wherein a distal end of the support section is connected to the connector assembly;
a clasp which is provided on the capture section and configured to clamp and fasten a predetermined target; and
a compression ring, a proximal end of the compression ring connected to the spacing portion and a distal end of the compression ring connected to the connector assembly,
wherein the prosthesis holder is configured to be clampingly coupled to a proximal end of the spacer by deploying the prosthetic members into the first deflected configuration and to be separated from the proximal end of the spacer by deploying the prosthetic members into the second deflected configuration.

12. The valve repair system according to claim 11, wherein the valve clip comprises a proximal connector and a sealing gasket provided on the proximal connector, wherein the spacer is clampingly coupled to the prosthetic members by the proximal connector and the sealing gasket.

13. The valve repair system according to claim 11, wherein the valve clip further comprises a support body, the support body disposed within the spacer and fixed near a distal end of the spacer, the support body defining a first guide bore that runs along the axis of the spacer,
the first guide bore comprising a first bore section, a second bore section and a third bore section, which are joined in order from proximal to distal, both the first bore section and the third bore section having a greater radial dimension than the second bore section, the radial dimension of the first bore section gradually decreasing from proximal to distal, the radial dimension of the third bore section gradually increasing from proximal to distal.

14. The valve repair system according to claim 11, wherein the clasp comprises a clasping sheet and a support sheet, the support sheet provided on the capture section, the clasping sheet elastically joined at one end to the support sheet so that the clasping sheet is elastically biased toward the support sheet.

15. The valve repair system according to claim 14, wherein the clasping sheet is provided with barbs including long barbs and short barbs, each long barb comprising a fixed portion, an intermediate portion and a tapered portion, which are joined in this order, the fixed portion connected to the clasping sheet.

16. The valve repair system according to claim 15, wherein the tapered portion is shaped as a quadrangular pyramid.

17. The valve repair system according to claim 14, wherein the clasping sheet is telescopic in an extension direction of the clasping sheet.

18. The valve repair system according to claim 17, wherein the clasping sheet comprises a plurality of telescopic sections, which are joined in the extension direction of the clasping sheet, the telescopic sections appear like waves.
